# EUROPEAN PATENT SPECIFICATION

(11) **EP 1 566 146 B1**
(45) Date of publication and mention of the grant of the patent: **04.04.2007**
(21) Application number: 05250950.2
(22) Date of filing: 18.02.2005
(51) Int. Cl.: A61B 10/00

(54) **Devices for extracting bodily fluids**
Geräte zur Entnahme von Körperflüssigkeiten
Outils pour l'extraction des liquides corporels

(30) Priority: 19.02.2004 US 783289
(43) Date of publication of application: 24.08.2005
(73) Proprietor: LifeScan, Inc., Milpitas, CA 95035 (US)
(72) Inventor: Hilgers, Michael Edward, Lake Elmo MN 55042 (US); McAllister, Devin V., Shrewbury MA 01545 (US); Menendez, Adolfo Jr, Cottage Grove MN 55016 (US)
(74) Representative: Mercer, Christopher Paul

(56) References cited:
- WO-A-02/100275
- US-A- 5 295 994
- US-A1- 2003 130 626
- US-B1- 6 319 210

## Description

### BACKGROUND OF INVENTION

### 1. Field of the Invention

The present invention relates, in general, to devices and methods for extracting bodily fluid and, in particular, to devices and methods that promote bodily fluid flow during extraction of the bodily fluid in a continuous or semi-continuous manner.

### 2. Description of the Related Art

In recent years, efforts in medical devices for monitoring the concentration of analytes (e.g., glucose) in bodily fluids (e.g., blood and interstitial fluid) have been directed toward developing devices and methods that allow continuous or semi-continuous monitoring. WO 02 100275 discloses such a device.

In the context of blood glucose monitoring, continuous or semi-continuous monitoring devices and methods are advantageous in that they provide enhanced insight into blood glucose concentration trends, the effect of food and medication on blood glucose concentration and a user's overall glycemic control. In practice, however, continuous and semi-continuous monitoring devices can have drawbacks. For example, during extraction of an interstitial fluid (ISF) sample from a target site (e.g., a user's dermal tissue target site) via a sampling module of a medical device, ISF quantities and/or flow rates can be insufficient and a user may experience discomfort.

Furthermore, continuous and semi-continuous monitoring devices can suffer from a deleterious effect known as "sensor lag." Such a sensor lag effect occurs when a significant difference exists between an analyte concentration at a sensor of the continuous monitoring device and the real-time analyte concentration at the target site.

Still needed in the field, therefore, is a device and associated method for extracting bodily fluid (such as ISF) that facilitate continuous or semi-continuous monitoring of the extracted bodily fluid while enhancing bodily fluid flow rate and/or quantity and/or reducing sensor lag effect. In addition, the device and associated method should reduce user discomfort.

### SUMMARY OF INVENTION

Embodiments of bodily fluid extraction devices and methods according to the present invention facilitate continuous or semi-continuous monitoring of extracted bodily fluid while enhancing bodily fluid flow rate and/or quantity and/or reducing sensor lag effect. In addition, the device and associated method can reduce user discomfort.

A bodily fluid extraction device according to an exemplary embodiment of the present invention includes a penetration member configured for penetrating a target site (such as a dermal tissue target site) and subsequently residing within the target site and extracting a bodily fluid sample. The penetration member includes a proximal end adapted for fluid communication with an analyte analysis system, a distal end, and a channel extending from the distal to the proximal end. The distal end includes a sharp portion for penetrating the target site and a flexible feature (e.g., a "leaf spring" flexible feature or an expandable flexible feature) adapted for promoting bodily fluid flow into the channel by protruding into the target site after the penetration member has penetrated the target site.

The bodily fluid extraction device optionally includes a flexible conduit for communicating an extracted sample from the channel to an analyte analysis system.

It is postulated, without being bound, that the flexible feature of embodiments of the present invention promotes bodily fluid flow by exerting a pressure on the target site, stretching the target site and/or creating a pocket within the target site. In addition, the use of a flexible conduit to communicate a bodily fluid sample from the penetration member to an analyte analysis module is expected to eliminate or reduce the discomfort that may otherwise result from the use of a completely stiff or rigid conduit.

A method of use includes providing a bodily fluid extraction device as described immediately above. Subsequently, a target site is penetrated with the distal end of the bodily fluid extraction device's penetration member and the flexible portion of the penetration member is caused to protrude into the target site and promote bodily fluid flow into the penetration member's channel. Bodily fluid (e.g., ISF) is then extracted from the target site (e.g., a dermal tissue target site) via the channel of the bodily fluid extraction device.

### BRIEF DESCRIPTION OF DRAWINGS

A better understanding of the features and advantages of the present invention will be obtained by reference to the following detailed description that sets forth illustrative embodiments, in which the principles of the invention are utilized, and the accompanying drawings of which:
FIG. 1A is a simplified perspective view of a bodily fluid extraction device according to an exemplary embodiment of the present invention;
FIG. 1B is a simplified cross-sectional side view of the bodily fluid extraction device of FIG. 1A;
FIG. 2 is a simplified cross-sectional side view of the bodily fluid extraction device of FIG. 1A residing in a dermal tissue target site;
FIGs. 3A and 3B are simplified perspective and cross-sectional views, respectively, of a bodily fluid extraction device according to another exemplary embodiment of the present invention;
FIGs. 4A and 4B are simplified perspective and cross-sectional views, respectively, of the bodily fluid extraction device of FIGs. 3A and 3B with the device's flexible feature in an expanded state;
FIG. 5 is a flow diagram illustrating a sequence of steps in a process according to an exemplary embodiment of the present invention;
FIG. 6A is a simplified perspective view of a bodily fluid extraction device according to an embodiment of the present invention; and
FIG. 6B is a simplified cross-sectional view of the bodily fluid extraction device of FIG. 6A.

### DETAILED DESCRIPTION OF THE INVENTION

For purposes of illustration throughout the following description, embodiments of the present invention will be described with reference to the extraction of interstitial fluid (ISF) for analyte (e.g., glucose) testing using a bodily fluid extraction device that penetrates into a dermal tissue target site. However, once apprised of the present disclosure one skilled in the art will recognize that embodiments of the present invention are not necessarily limited to the extraction of ISF.

Embodiments of the present invention can be included, for example, in ISF extraction devices that are adapted to provide continuous or semi-continuous flow of ISF to an analyte monitoring device. Examples of ISF extraction devices with which embodiments of the present invention may be used are described in WO 02/49507 A1 and U.S. Patent Application No. 10/653,023 (US 2004 249 253). Examples of combined sample collection and metering systems designed for *in-situ* testing as can also be used with embodiments of the present invention are described in WO 01/64105 and WO 2003/015627 and EP-A-1 360 931.

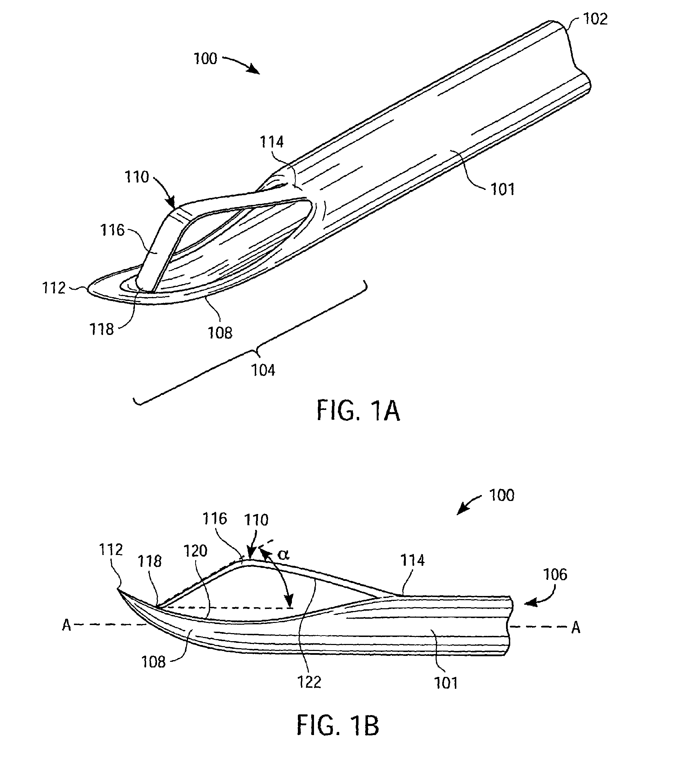

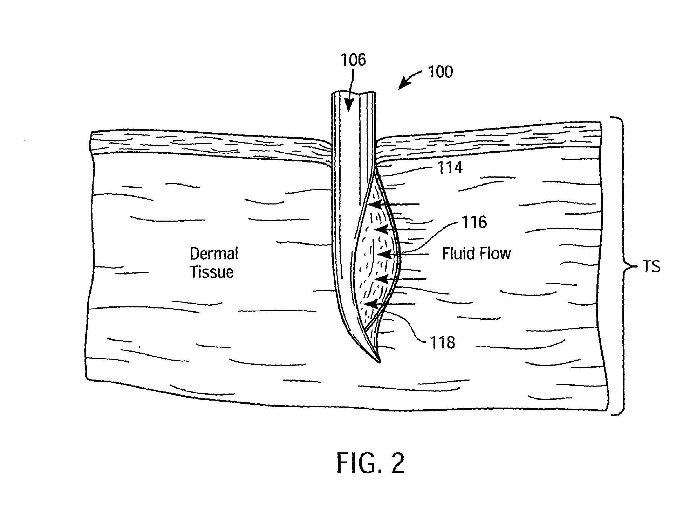

FIGs. 1A and 1B are perspective and cross-sectional side views, respectively, of a bodily fluid extraction device 100 (e.g., an ISF extraction device) according to an exemplary embodiment of the present invention. FIG. 2 is a cross-sectional depiction of bodily fluid extraction device 100 residing within a dermal tissue target site.

Referring to FIGs. 1A, 1B and 2, bodily fluid extraction device 100 is configured for penetrating a target site (e.g., the dermal tissue target site depicted in FIG. 2) and subsequently residing within the target site and extracting a bodily fluid sample therefrom. Bodily fluid extraction device 100 includes a penetration member 101. In the embodiment of FIGs. 1A, 1B and 2, bodily fluid extraction device 100 is depicted as including solely penetration member 101. However, as described below, bodily fluid extraction devices according to embodiments of the present invention can include additional components, such as a flexible conduit.

Penetration member 101 includes a proximal end 102 adapted for fluid communication with an analyte analysis system (not shown), a distal end 104 and a channel 106 (such as a channel with an inner diameter in the range of 0.25 mm to 1.25 mm) extending from the distal end to the proximal end. The walls of channel 106 can have a thickness in the range, for example, of about 0.02 mm to about 0.5 mm. In addition, the length of penetration member 101 is such that it can reside in a dermal tissue target site to a maximum depth in the range of from about 1.5 mm to 3 mm below the surface of the dermal tissue target site.

Proximal end 102 is configured to be in fluid communication with an analyte analysis system (not shown), an example of which is described in the aforementioned WO 02/49507 A1 and U.S. Patent Application No. 10/653,023 (US 2004 249 253). Proximal end 102 can, if desired, communicate with such an analyte analysis system via a flexible conduit of appropriate inner diameter.

Distal end 104 includes a sharp portion 108 for penetrating a target site, and a flexible feature 110 adapted for promoting bodily fluid flow into channel 106 by protruding into the target site either during penetration or after the penetration member has penetrated the target site. In the embodiment of FIGs. 1A, 1B and 2, sharp portion 108 has a sharp tip 112 to facilitate penetration of a target site.

Flexible feature 110 is configured as a "leaf spring" in a manner that provides for flexible feature 110 to be deflected and/or compressed as penetration member 101 penetrates a target site (e.g., as the distal end penetrates dermal tissue or subcutaneous tissue). Such deflection (and/or compression) causes the flexible feature to protrude into, and exert pressure on, the target site, thus promoting the flow of bodily fluid from the target site into channel 106. In this respect, flexible feature 110 serves as a target site displacement member. It is also postulated, without being bound, that the flexible nature of the flexible feature improves the ease by which the penetration member can penetrate a target site in comparison to a theoretical rigid penetration member that is capable of displacing an equivalent volume in the target site.

Flexible feature 110 includes a first end 114, an arch 116 and a second end 118. Second end 118 can be, for example, rounded or tapered to a point. Second end 118 is a "free end" in that second end 118 is not attached to any portion of the bodily fluid extraction device other than arch 116. A tangential line to arch 116 from second end 118 (in a plane of symmetry of flexible feature 110) defines angle α with a longitudinal axis A-A of channel 106 (see FIG. 1B). Angle α typically is in the range of about 1 degree to 75 degrees prior to deflection or compression of flexible feature 110.

Once apprised of the present disclosure, those skilled in the art will recognize that angle α will affect the manner in which flexible feature 110 and penetration member 101 interact with the target site both during and after penetration. The initial value (i.e., the value prior to penetration) for angle α is typically in the range of from about 1 to about 75 degrees. The final value (i.e., the value after the penetration member has penetrated the target site and the flexible feature caused to protrude) for angle α ranges from about 5 to about 80 degrees. The change in angle α before and after insertion of the penetration member is typically, for example, in the range of about 5 degrees to about 75 degrees. Angle α can either increase such that arch 116 protrudes into and exerts pressure on the target site or angle α can decrease such that second end 118 protrudes into and exerts pressure on the target site.

Bodily fluid extraction device 100 can be formed of, for example, a non-corroding metal (e.g., stainless steel) or other non-corroding material, such as a plastic material. Bodily fluid extraction device 100 can be formed using, for example, conventional metal stamping, sheet metal forming, micro-machining, welding and polymer molding techniques.

The bodily fluid extraction performance of bodily fluid extraction devices according to embodiments of the present invention can be enhanced by electrical, mechanical, chemical, or other methods or combinations thereof that serve to beneficially decrease surface roughness and/or by the application of coatings that inhibit bodily fluid clotting (e.g., a heparin-based coating).

Although flexible feature 110 is depicted in FIGs. 1A, 1B and 2 as a solid component for illustration purposes, it can optionally include pores (i.e., openings) throughout. The thickness of flexible feature 110 should be sufficient to ensure mechanical stability while maintaining flexibility. For example, the thickness of flexible feature 110 can be in the range of about 0.02 mm to about 0.50 mm. Flexible feature 110 can be, for example, of a uniform width or tapered gradually from first end 114 to second end 118.

When bodily fluid extraction device 100 is employed to penetrate a dermal tissue target site, penetration member 101 and flexible feature 110 penetrate the target site, as shown in FIG. 2. As the penetration member 101 is urged into the dermal tissue target site, flexible feature 110 flexes such that, for example, second end 118 moves closer to first end 114. In this circumstance, the flexing of flexible feature 110 causes angle α to increase. This results in flexible feature 110 protruding outward into the dermal tissue. As flexible feature 110 protrudes (with either an increasing or decreasing angle α) pressure is exerted on the tissue and a 'tissue pocket' is formed, forcing bodily fluid (e.g., ISF) out of the dermal tissue and into channel 106 (see FIG. 2 where arrows indicate the direction of bodily fluid flow). Bodily fluid (e.g., ISF) can also flow through the pores, if optionally present, in flexible feature 110. A typical impact velocity required to insert the penetration member into a human dermal tissue target site and flex the flexible feature is, for example, in the range of 1 to 6 meters per second.

A result of the flexing of flexible feature 110 is the promotion (e.g., an increase in flow rate and/or flow quantity) of ISF flow from the dermal tissue target site. An increased ISF flow rate is desirable because it can reduce sensor lag. Yet another benefit of an increased ISF flow rate is that more analytical measurements can be taken in a given period of time. A benefit of increased flow quantity is the simplification that comes from handling and analyzing larger volumes.

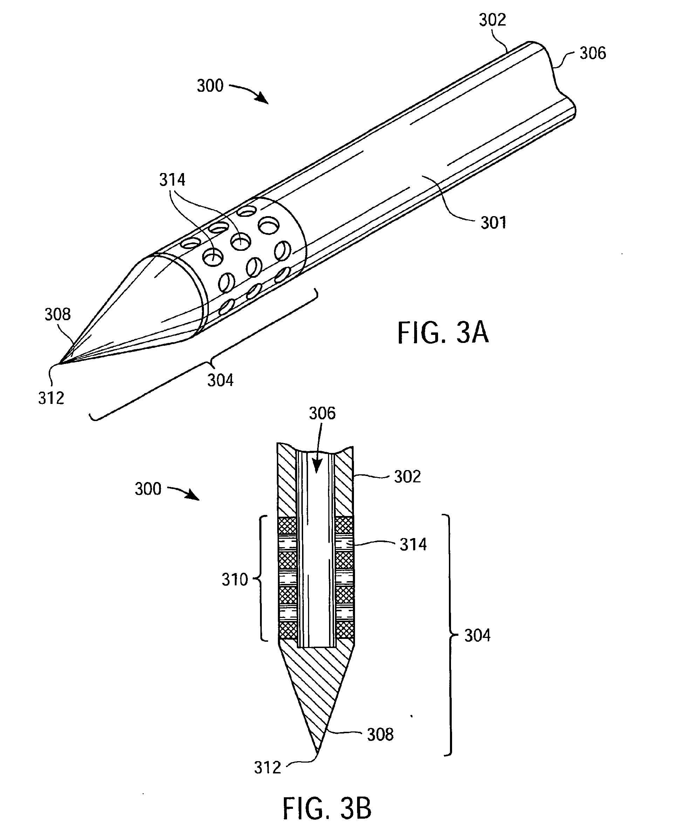

FIGs. 3A and 3B are simplified perspective and cross-sectional views, respectively, of a bodily fluid extraction device 300 according to another exemplary embodiment of the present invention. In the embodiment of FIGs. 3A and 3B, bodily fluid extraction device 300 is depicted as including solely penetration member 301. However, as described below, bodily fluid extraction device 300 can include additional components, such as a flexible conduit.

Penetration member 301 includes a proximal end 302 adapted for fluid communication with an analyte analysis system (not shown), a distal end 304 and a channel 306 extending from the distal end to the proximal end.

Proximal end 302 is configured to be in fluid communication with an analyte analysis system (not shown), an example of which is described in the aforementioned WO 02/49507 A1 and U.S. Patent Application No. 10/653,023 (US 2004 249 253). Proximal end 302 can communicate with such an analyte analysis system via a flexible conduit of appropriate inner diameter.

Distal end 304 includes a sharp portion 308 for penetrating a target site, and a flexible feature 310 adapted for promoting bodily fluid flow into channel 306 by protruding into the target site after the penetration member has penetrated the target site. In the embodiment of FIGs. 3A and 3B, sharp portion 308 has a sharp tip 312 to facilitate penetration of a target site and flexible feature 310 includes pores (i.e., openings) 314 therethrough. Pores 314 provide a means for bodily fluid to travel from a target site that has been penetrated to channel 306.

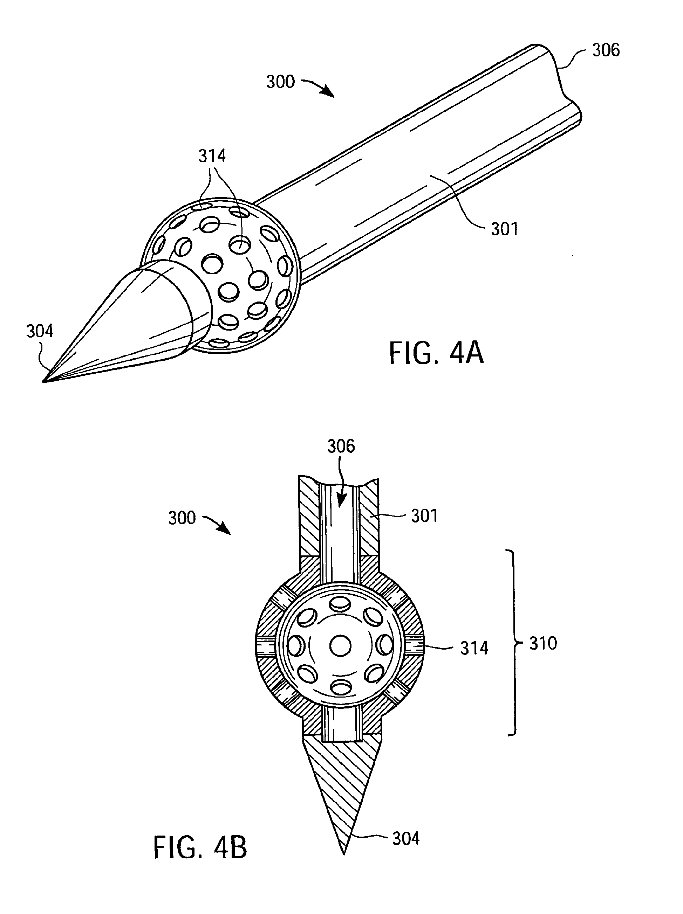

FIGs. 4A and 4B are simplified perspective and cross-sectional views, respectively, of bodily fluid extraction device 300 with the device's flexible feature 310 in an expanded state. For simplification, FIGs. 4A and 4B do not depict a target site. However, it should be understood that, when in an expanded state, flexible feature 310 protrudes into a target site and exerts pressure thereon to promote bodily fluid flow from the target site into the penetration member via pores 314. Flexible feature 310 can be placed into an expanded state after flexible feature 310 has penetrated a target site using, for example, mechanical (e.g., spring) displacement, fluid pressurization or nitinol activation techniques. In this regard, the flexible feature can be formed at least partially of, for example, a hydrogel material that swells when it comes into contact with bodily fluid, a nitinol material that expands upon the application of an electric current or a expandable balloon.

In FIGs. 4A and 4B, flexible feature 310 is shown as having a spherical shape when in the expanded state. It should be noted however, that the flexible feature can take other shapes when in the expanded state, including but not limited to, shapes that include a portion with an elliptical cross-section.

Pores 314 are of a suitable size such that the pores are not clogged by bodily fluid components during use. A typical range for the diameter of pores 314 is from approximately 5 micrometers to approximately 0.5 millimeters. The porosity of flexible feature 310 due to the presence of pores 314 can be, for example, in the range of from about 1% to about 95%. If the porosity percentage is too low, then an optimally high bodily fluid flow rate may not be achieved. If the percentage is too high, the physical integrity of the flexible feature may be compromised.

Depending on the type of analyte measurement to be performed, the bodily fluid extraction devices illustrated in FIGs. 1A-4B can reside in a dermal tissue target site for extended periods of time, such as an extended time period in the range of 30 minutes to 72 hours. In addition, those skilled in the art will recognize that embodiments of bodily fluid extraction devices according to the present invention can be used as penetration members in conjunction with extraction devices that include pressure ring(s), an example of which is illustrated and described in U.S. Patent No. 5,682,233. Embodiments of bodily fluid extraction devices according to the present invention can also be employed in conjunction with an extraction device containing one or more oscillatible pressure rings as described in U.S. Patent Application No. 10/653,023 (US US 2004 249 253).

Those skilled in the art will recognize that bodily fluid extraction devices according to embodiments of the present invention can also be used in combination with other techniques that enhance ISF flow rate including, but not limited to, application of a low current to the target site, application of heat to the target site and/or to the penetration member, application of a vacuum to the target site or vacuum applied to channel of the penetration member.

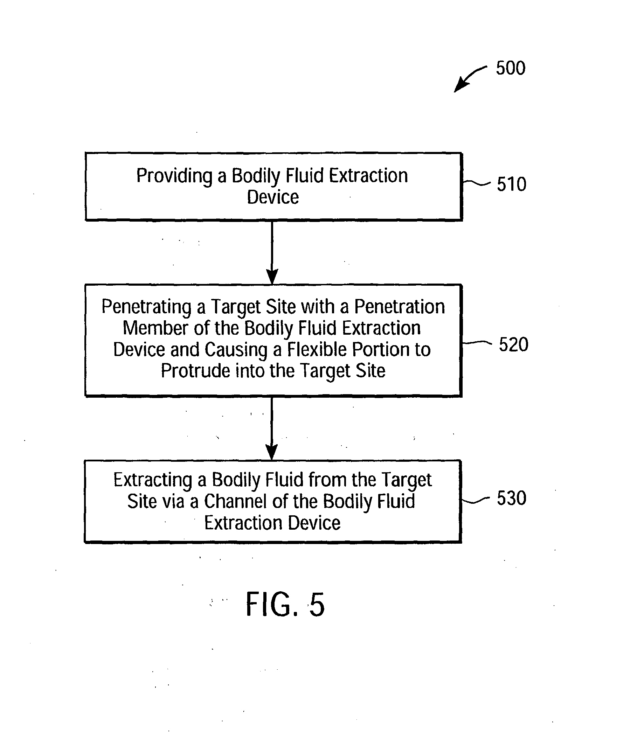

Referring to FIG. 5, a method 500 for collection of ISF from a target site according to an exemplary embodiment of the present invention includes providing a bodily fluid extraction device, as set forth in step 510. The bodily fluid extraction device includes a penetration member configured for penetrating a target site (e.g., a dermal tissue target site) and subsequently residing within the target site and extracting a bodily fluid sample therefrom. The penetration member includes a proximal end adapted for fluid communication with an analyte analysis system, a distal end, and a channel extending from the distal end to the proximal end. In addition, the distal end includes a sharp portion for penetrating the target site and a flexible feature adapted for promoting bodily fluid flow into the channel by protruding into the target site after the penetration member has penetrated the target site.

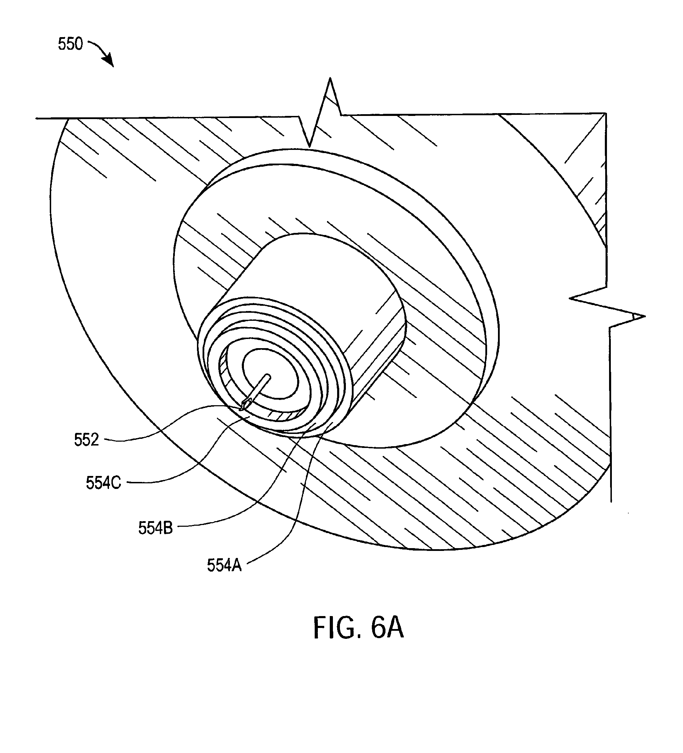

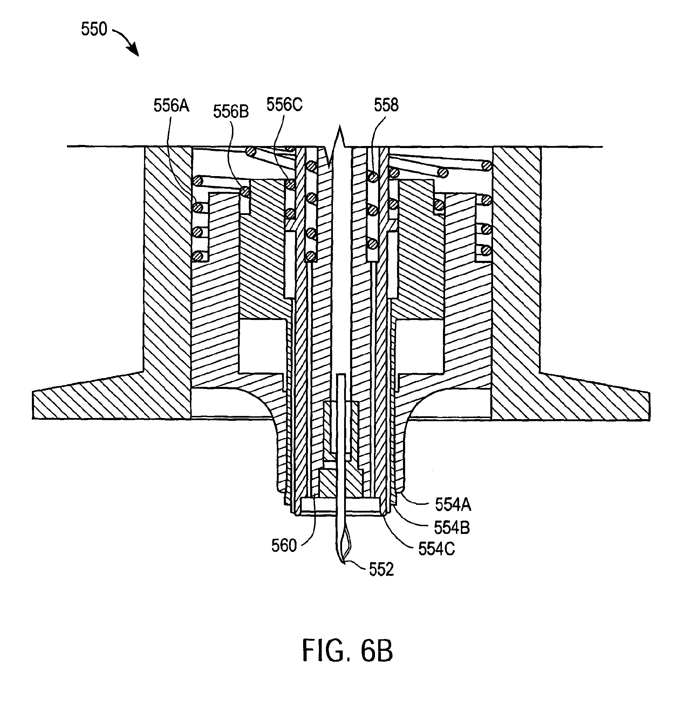

Subsequently, as set forth in step 520, the target site is penetrated with the distal end of the penetration member and the flexible portion is caused to protrude into the target site and promote bodily fluid flow into the channel. The flexible feature can be caused to protrude while the penetration member is penetrating the target site and/or after the penetration member has penetrated the target site. Bodily fluid (e.g., ISF) is then extracted from the target site via the channel of the bodily fluid extraction device, as set forth in step 530.As previously described, embodiments of bodily fluid extraction devices according to the present invention can also be employed in conjunction with an extraction device containing one or more oscillatable pressure rings. FIGS. 6A and 6B are simplified perspective and cross-sectional side views, respectively, of a bodily fluid extraction device 550 according to yet another exemplary embodiment of the present invention. Bodily fluid extraction device 550 includes a bodily fluid extraction member 552 (i.e., penetration member 101 of FIG. 1) and a plurality of concentrically arranged oscillatible pressure rings 554A, 554B and 554C. ISF extraction device 550 also includes a plurality of first biasing elements 556A, 556B and 556C for urging the pressure rings 554A, 554B and 556C, respectively, toward and against a user's skin layer (i.e., a dermal tissue target site), a second biasing element 558 for launching bodily fluid extraction member 552, and a penetration depth control element 560.

During use, bodily fluid extraction device 550 is positioned such that pressure rings 554A, 554B and 554C are facing a user's skin layer. This can be accomplished, for example, by employing bodily fluid extraction device 550 in a sampling module of a system for extracting bodily fluid (as described in the aforementioned WO 02/49507 and EP-A-1 491 144) and placing the system against the user's skin layer.

Pressure ring 554A is then urged against the user's skin layer by first biasing element 556A, thereby creating a bulge in the user's skin layer that will subsequently be lanced (i.e., penetrated) by bodily fluid extraction member 552. While pressure ring 554A is in use (i.e., deployed), pressure ring 554B and pressure ring 554C can be maintained in a retracted position by first biasing elements 556B and 556C, respectively.

Bodily fluid (e.g., ISF) can be extracted from the bulge formed in the user's skin layer while bodily fluid extraction member 552 resides totally or partially within the user's skin layer. After about 3 seconds to 3 hours, the pressure ring 554A can be retracted to allow the user's skin layer to recover for a time period in the range of about 3 seconds to 3 hours. After retracting the pressure ring 554A, pressure ring 554B can be deployed to apply pressure on the user's skin layer. While pressure ring 554B is in use (i.e., deployed), pressure ring 554A and pressure ring 554C can be maintained in a retracted position by first biasing elements 556A and 556C, respectively. After a time period of about 3 seconds to 3 hours, pressure ring 554B can be retracted for a time period in the range of 3 seconds to 3 hours, followed by the deployment of pressure ring 554C. Pressure ring 554C maintains pressure on the user's skin layer for a time period in the range of 3 seconds to 3 hours and is then retracted for a time period in the range of 3 seconds to 3 hours. While pressure ring 554C is in use (i.e., deployed), pressure ring 554A and pressure ring 554B can be maintained in a retracted position by first biasing elements 556A and 556B, respectively. This process of cycling between deployment and retraction of pressure rings 554A, 554B and 554C (i.e., the oscillation of the pressure rings 554A, 554B and 554C) can proceeds until fluid extraction has ended. The deployment and retraction cycles in the multiple pressure ring embodiment of FIGs. 6A and 6B are preferably asymmetric in that different periods of time are used for each cycle but need not necessarily be so.

Those skilled in the art will also recognize that a plurality of pressure rings in bodily fluid extraction devices according to embodiments of the present invention can be deployed in any order and that one is not limited to the deployment and retraction sequence described above. For example, a sequence can be used in which pressure ring 554B or 554C is applied before pressure ring 554A. Further, more than one pressure ring can be deployed simultaneously. For example, the embodiment shown in FIGs. 6A and 6B can deploy all three pressure rings simultaneously such that the pressure rings function as a single pressure ring.

For the embodiment shown in FIGs. 6A and 6B, the pressure applied against the user's skin can, for example, range from about 0.1 to 150 pounds per square inch (PSI) for each of the plurality of pressure rings.

The pressure rings 554A, 554B and 554C can have, for example, outer diameters of in the range of 0.08 to 0.560 inches, 0.1 to 0.9 inches and 0.16 to 0.96 inches, respectively. The wall thickness of each pressure ring can be, for example, in the range of 0.02 to 0.04 inches.

Inclusion of at least one pressure ring in extraction devices according to embodiments of the present invention provides a number of benefits for extraction of ISF. First, oscillating the pressure ring(s) between a deployed and retracted state serves to mitigate (i.e., reduce) ISF glucose lag. Upon retraction of the pressure ring(s), pressure on the user's skin layer is released, and the user's body reacts by increasing blood perfusion to the target site. This phenomenon is known as reactive hyperemia and is hypothesized to be a mechanism by which ISF is beneficially replenished in the target site by oscillation of the pressure ring(s). Such a replenishment of ISF helps mitigating the lag between the ISF glucose and whole blood glucose values.

Another benefit of bodily fluid extraction devices according to the present invention is that oscillation of the pressure ring(s) allows the skin under the pressure ring(s) to recover, thus reducing a user's pain, discomfort and the creation of persistent blemishes.

Moreover, extraction devices with a plurality of pressure rings (e.g., the embodiment of FIGs. 6A and 6B) can be used with at least one pressure ring permanently deployed to facilitate ISF collection while the other pressure rings are oscillated between deployed and retracted states so that different areas of the user's skin layer are under pressure at any given time. Such combination of permanently deployed pressure ring(s) and oscillated pressure ring(s) further aids in reducing a user's pain/discomfort.

Still another benefit of bodily fluid extraction devices according to embodiments of the present embodiment is that the pressure ring(s) can be used in conjunction with the bodily fluid extraction member to increase the flow rate of ISF extracted from a lance site even more than when only the bodily fluid extraction member is used to extract ISF from a lance site. Increased ISF flow rate is desirable because it reduces the lag in time between the glucose concentration measured in ISF and the actual glucose concentration obtained from whole blood. Another benefit of an increased ISF flow rate is that greater sample volumes of ISF are available for testing at any given time. Thus, a glucose detection system used in conjunction with the present invention is easier to develop and manufacture than are systems that must utilize very small volumes of ISF. Yet another benefit of an increased ISF flow rate is that more measurements can be taken in a given period of time.

While preferred embodiments of the present invention have been shown and described herein, those skilled in the art will recognize that such embodiments are provided by way of example only. Numerous variations, changes and substitutions will occur to those skilled in the art without departing from the invention.

It should be understood that various alternatives to the embodiments of the invention described herein may be employed in practising the invention. It is intended that the following claims define the scope of the invention and that methods and structures within the scope of these claims and their equivalents be covered thereby.

## Claims

1. A bodily fluid extraction device (100) comprising:
a penetration member (101) configured for penetrating a target site and subsequently residing within the target site and extracting a bodily fluid sample therefrom, the penetration member including:
a proximal end (102) adapted for fluid communication with an analyte analysis system;
a distal end (104); and
a channel (106) extending from the distal end to the proximal end,
wherein the distal end includes:
a sharp portion (108) for penetrating the target site; **characterized in that** the distal end further includes
a flexible feature (110,310) adapted for promoting bodily fluid flow into the channel by protruding into the target site after the penetration member (101) has penetrated the target site.

2. The bodily fluid extraction device of claim 1 further including a conduit attached to the proximal end of the penetration member and configured for transporting an extracted bodily fluid sample from the penetration member to an analyte analysis module.

3. The bodily fluid extraction device of claim 2, wherein the conduit is made of a flexible material.

4. The bodily fluid extraction device of any one of claims 1 to 3, wherein the flexible feature includes:
a first end (114);
a free second end (118); and
an arch (116) between the first end (114) and the second end (118).

5. The bodily fluid extraction device of claim 4, wherein the flexible feature is configured as a leaf spring.

6. The bodily fluid extraction device of any one of claims I to 5, wherein the flexible feature is an expandable flexible feature.

7. The bodily fluid extraction device of claim 6, wherein the flexible feature includes pores (314).

8. The bodily fluid extraction device of any one of claims 1 to 7, wherein the flexible feature is adapted for promoting interstitial fluid flow into the channel (106).

9. The bodily fluid extraction device of any one of claims 1 to 8 further including at least one pressure ring (554).

10. The bodily fluid extraction device of claim 9, wherein the at least one pressure ring (554) is a plurality of oscillatible pressure rings (554 A,B,C).

11. The bodily fluid extraction device of any one of claims 1 to 10 for use in a method for extracting bodily fluid by providing said device, penetrating a target site with the distal end of the penetration member, causing the flexible portion to protrude into the target site and promote bodily fluid flow into the channel; and extracting bodily fluid from the target site via the channel of the bodily fluid extraction device.

## Patentansprüche

1. Körperfluidextraktionsvorrichtung (100), welche umfaßt:
ein Penetrationselement (101), das dazu konfiguriert ist, zu einem Zielort vorzudringen und nachfolgend am Zielort zu bleiben und eine Körperfluidprobe
davon zu extrahieren, wobei das Penetrationselement umfaßt:
ein proximales Ende (102), das für eine Fluidkommunikation mit einem Analyt-Analysesystem eingerichtet ist;
ein distales Ende (104); und
einen Kanal (106), der sich vom distalen zum proximalen Ende erstreckt, wobei das distale Ende umfaßt:
einen scharfen Abschnitt (108) zum Vordringen zum Zielort; **dadurch gekennzeichnet, daß** das distale Ende des weiteren ein flexibles Merkmal (110, 310) umfaßt, das dazu eingerichtet ist, einen Körperfluidfluß in den Kanal durch Vorragen in den Zielort zu fördern, nachdem das Penetrationselement 102 zum Zielort vorgedrungen ist.

2. Körperfluidextraktionsvorrichtung nach Anspruch 1, die des weiteren eine Leitung umfaßt, die am proximalen Ende des Penetrationselements befestigt ist und zum Transportieren einer extrahierten Körperfluidprobe vom Penetrationselement zum Analyt-Analysemodul konfiguriert ist.

3. Körperfluidextraktionsvorrichtung nach Anspruch 2, wobei die Leitung aus einem flexiblen Material hergestellt ist.

4. Körperfluidextraktionsvorrichtung nach einem der Ansprüche 1 bis 3, wobei das flexible Merkmal umfaßt:
ein erstes Ende (114);
ein freies zweites Ende (118) und
einen Bogen (116) zwischen dem ersten (114) und zweiten Ende (118).

5. Körperfluidextraktionsvorrichtung nach Anspruch 4 wobei das flexible Merkmal als eine Blattfeder konfiguriert ist.

6. Körperfluidextraktionsvorrichtung nach einem der Ansprüche 1 bis 5, wobei das flexible Merkmal ein erweiterbares flexibles Merkmal ist.

7. Körperfluidextraktionsvorrichtung nach Anspruch 6, wobei das flexible Merkmal Poren (314) umfaßt.

8. Körperfluidextraktionsvorrichtung nach einem der Ansprüche 1 bis 7, wobei das flexible Merkmal dazu eingerichtet ist, einen interstitiellen Fluidfluß in den Kanal (106) zu fördern.

9. Körperfluidextraktionsvorrichtung nach einem der Ansprüche 1 bis 8, die des weiteren zumindest einen Druckring (554) umfaßt.

10. Körperfluidextraktionsvorrichtung nach Anspruch 9, wobei der zumindest eine Druckring (554) mehrere oszillierbare Druckringe (554A, B, C) umfaßt.

11. Körperfluidextraktionsvorrichtung nach einem der Ansprüche 1 bis 10 zur Verwendung in einem Verfahren zum Extrahieren von Körperfluid durch Bereitstellen der Vorrichtung, Vordringen zum Zielort mit dem distalen Ende des Penetrationselements, Bewirken, daß der flexible Abschnitt in den Zielort vorragt und Fördern eines Körperfluidflusses in den Kanal und Extrahieren von Körperfluid vom Zielort über den Kanal der Körperfluidextraktionsvorrichtung.

## Revendications

1. Dispositif pour l'extraction de fluide corporel (100) comprenant :
un élément de pénétration (101) configuré pour pénétrer dans un site cible et résider par la suite à l'intérieur du site cible et extraire un échantillon de fluide corporel de celui-ci, l'élément de pénétration comprenant :
une extrémité proximale (102) adaptée pour une communication fluidique avec un système d'analyse d'analyte ;
une extrémité distale (104) ; et
un canal (106) s'étendant depuis l'extrémité distale vers l'extrémité proximale,
dans lequel l'extrémité distale comprend:
une partie pointue (108) pour pénétrer dans le site cible ; **caractérisé en ce que** l'extrémité distale comprend en outre
un élément flexible (110, 310) adapté pour promouvoir l'écoulement de fluide corporel à l'intérieur du canal en faisant saillie à l'intérieur du site cible après que l'élément de pénétration (101) ait pénétré dans le site cible.

2. Dispositif pour l'extraction de fluide corporel selon la revendication 1 comprenant en outre un conduit fixé à l'extrémité proximale de l'élément de pénétration et configuré pour transporter un échantillon de fluide corporel extrait depuis l'élément de pénétration vers un module d'analyse d'analyte.

3. Dispositif pour l'extraction de fluide corporel selon la revendication 2, dans lequel le conduit est fabriqué dans un matériau flexible.

4. Dispositif pour l'extraction de fluide corporel selon l'une quelconque des revendications 1 à 3, dans lequel l'élément flexible comprend :
une première extrémité (114) ;
une seconde extrémité libre (118) ; et
un arc (116) entre la première extrémité (114) et la seconde extrémité (118).

5. Dispositif pour l'extraction de fluide corporel selon la revendication 4, dans lequel l'élément flexible est configuré comme un ressort à lame.

6. Dispositif pour l'extraction de fluide corporel selon l'une quelconque des revendications 1 à 5, dans lequel l'élément flexible est un élément flexible extensible.

7. Dispositif pour l'extraction de fluide corporel selon la revendication 6, dans lequel l'élément flexible comprend des pores (314).

8. Dispositif pour l'extraction de fluide corporel selon l'une quelconque des revendications 1 à 7, dans lequel l'élément flexible est adapté pour promouvoir l'écoulement de fluide interstitiel à l'intérieur du canal (106).

9. Dispositif pour l'extraction de fluide corporel selon l'une quelconque des revendications 1 à 8 comprenant en outre au moins un anneau de pression (554).

10. Dispositif pour l'extraction de fluide corporel selon la revendication 9, dans lequel l'au moins un anneau de pression (554) est une pluralité d'anneaux de pression pouvant osciller (554 A, B, C).

11. Dispositif pour l'extraction de fluide corporel selon l'une quelconque des revendications 1 à 10 à utiliser dans un procédé d'extraction de fluide corporel en fournissant ledit dispositif, en pénétrant dans un site cible avec l'extrémité distale de l'élément de pénétration, en faisant que la partie flexible fait saillie à l'intérieur du site cible et promeut l'écoulement de fluide corporel à l'intérieur du canal ; et en extrayant du fluide corporel du site cible via le canal du dispositif pour l'extraction de fluide corporel.
